# EUROPÄISCHE PATENTSCHRIFT

(11) **EP 1 959 998 B1**
(45) Veröffentlichungstag und Bekanntmachung des Hinweises auf die Patenterteilung: **03.03.2010**
(21) Anmeldenummer: 06830232.2
(22) Anmeldetag: 30.11.2006
(51) Int. Cl.: A61K 47/32, C08F 8/00

(54) **COPOLYMERE AUF BASIS VON POLYALKYLENOXID-MODIFIZIERTEN N-VINYLLACTAM-COPOLYMEREN**
COPOLYMERS BASED ON POLYALKYLENE OXIDE-MODIFIED N-VINYL LACTAM COPOLYMERS
COPOLYMERES A BASE DE COPOLYMERES DE N-VINYLLACTAME A MODIFICATION POLYALKYLENE-OXYDE

(30) Priorität: 09.12.2005 EP 05111921
(43) Veröffentlichungstag der Anmeldung: 27.08.2008
(73) Patentinhaber: BASF SE, 67056 Ludwigshafen (DE)
(72) Erfinder: BOUILLO, Nathalie, 76532 Baden-Baden (DE); KHVOROST, Alexander, 69514 Laudenbach (DE); MÜNSTER, Ingo, 67459 Böhl-Iggelheim (DE); DOBRAWA, Rainer, 68167 Mannheim (DE); MEYER-BÖHM, Kathrin, 68163 Mannheim (DE); LANGE, Ronald Frans Maria, 67061 Ludwigshafen (DE)
(86) Internationale Anmeldenummer: PCT/EP2006/069128
(87) Internationale Veröffentlichungsnummer: WO 2007/065845

(56) Entgegenhaltungen:
- EP-A2- 0 368 217
- EP-B1- 1 587 848
- US-A- 3 086 956
- US-A1- 2005 090 612

## Beschreibung

Die Erfindung betrifft mit Polyalkylenoxid-Seitenketten modifizierte N-Vinyllactam-Copolymeren, deren Herstellung und deren Verwendung als Solubilisatoren von in Wasser schwerlöslichen biologisch aktiven Substanzen. Weiterhin betrifft die Erfindung entsprechende Zubereitungen für die Anwendung an Mensch, Tier und Pflanze.

Bei der Herstellung homogener Zubereitungen biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solublisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt (Vgl. Römpp Chemie Lexikon, 9. Auflage, Bd.5. S. 4203, Thieme Verlag, Stuttgart, 1992).

Die hergestellten Solubilisate sind **dadurch gekennzeichnet, daß** der schlecht wasserlösliche oder wasserunlösliche Stoff in den Molekülassoziaten der oberflächenaktiven Verbindungen, die sich in-wäßriger Lösung bilden - den sogenannten Mizellen - kolloidal gelöst vorliegt. Die resultierenden Lösungen sind stabile einphasige Systeme, die optisch klar bis opaleszent erscheinen und ohne Energieeintrag hergestellt werden können.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich Tenside wie ethoxiliertes Ricinusöl oder ethoxiliertes hydriertes Ricinusöl, ethoxilierte Sorbitanfettsäureester oder ethoxilierte Hydroxystearinsäure eingesetzt.

Die oben beschriebenen, bisher eingesetzten Solubilisatoren zeigen jedoch eine Reihe anwendungstechnischer Nachteile.

Die bekannten Solubilisatoren besitzen für einige schwerlösliche Arzneistoffe wie z.B. Clotrimazol nur eine geringe lösungsvermittelnde Wirkung.

Weiterhin sind die bisher bekannten Solubilisatoren meist flüssige oder halbfeste Verbindungen, die aufgrund dessen ungünstigere Verarbeitungseigenschaften aufweisen.

US 4,432,881 beschreibt hydrophob modifizierte Polyacrylsäure mit einem Molekulargewicht zwischen 200000 und 5000000, die durch Copolymerisation von Acrylsäure mit den entsprechenden N-Alkylacrylamiden oder Acrylaten erhalten werden. Verwendet werden die erhaltenen Polymere als dispergierbare hydrophobe Verdicker.

In der US 4,395,524 wird die Copolymerisation von hydrophilen Komponenten (z.B. Acrylamid, Acrylsäure, N-Vinylpyrrolidon u.a.) mit N-Alkylacrylamiden beschrieben. Die so erhaltenen Polymere mit einem Molekulargewicht von 30000 bis 2000000 werden als Verdicker, Sedimentationsstabilisatoren oder Dispergiermittel verwendet.

EP-A-0 268 164 beschreibt die Verwendung von Copolymerisaten von monoolefinisch ungesättigten Säuren und Alkylester monoolefinisch ungesättigter Säuren zur Stabilisierung von O/W-Emulsionen.

In der EP-A 876 819 ist die Verwendung von Copolymeren aus mindestens 60 Gew.-% N-Vinylpyrrolidon und Amiden oder Estern mit langkettigen Alkylgruppen als Lösungsvermittler für schwerlösliche Wirkstoffe beschrieben.

In der EP-A 948 957 ist die Verwendung von Copolymerisaten aus monoethylenisch ungesättigten Carbonsäuren wie beispielsweise Acrylsäure und hydrophob modifizierten Comonomeren wie beispielsweise N-Alkyl- oder N,N-Dialkyl- Amiden ungesättigter Carbonsäuren mit C₈-C₃₀-Alkylresten als Lösungsvermittler für schwerlösliche Wirkstoffe beschrieben.

Es bestand die Aufgabe, neue Solubilisatoren für pharmazeutische, kosmetische, lebensmitteltechnische sowie agrotechnische Anwendungen bereitzustellen.

Diese Aufgabe wurde gelöst durch die eingangs definierten Copolymere, welche durch Umsetzung eines hydroxyl- oder amino-funktionalisierten N-Vinyllactam- Copolymeren mit Polyalkylenoxiden oder durch direkte Polyalkoxilierung der Hydroxyl- oder Aminogruppen des N-Vinyllactam-Copolymeren erhalten werden.

Weiterhin betrifft die Erfindung deren Verwendung als Solubilisatoren für in Wasser schwerlösliche Substanzen sowie entsprechende Zubereitungen.

Die als Präpolymere eingesetzten Vinyllactam-Copolymeren werden erhalten durch radikalisch initiierte Copolymerisation von 50 bis 99,9 mol.-% N-Vinyllactamen mit 0,1 bis 50 mol-% eines weiteren monoolefinisch ungesättigten Comonomeren. Bevorzugt werden 80 bis 99 mol-% N-Vinyllactam und 1 bis 20 mol-% des Comonomeren eingesetzt.

Als N-Vinyllactame eignen sich N-Vinylpyrrolidon, N-Vinylpiperidon und N-Vinylcaprolactam oder Mischungen dieser Monomere. Bevorzugtes N-Vinyllactam ist N-Vinylpyrrolidon.

Als Comonomere eignen sich monoolefinisch ungesättigte Monomere, die Hydroxyl-oder Amino-Gruppen tragen oder eine durch Hydrolyse in Hydroxyl- oder Amino-Gruppen überführbare Gruppe enthalten.

Geeignete Comonomere sind demnach:
Vinylester von C₂-C₈-Carbonsäuren wie Vinylacetat oder Vinylpropionat,
Monovinylether von C₂-C₈-Diolen, beispielsweise Ethylenglykolmonovinylether, Diethylengklykolmonovinylether, 1,4-Butandiolmonovinylether, 1,6-Hexandiolmonovinylether oder 1,4-Cyclohexandimethanolmonovinylether,
N-Vinylamide wie beispielsweise N-Vinylacetamid oder N-Vinylformamid,
Hydroxyalkylester oder Aminoalkylester der Acrylsäure oder der Methacrylsäure mit C₁-C₈-Alkylkettenlängen, wie beispielsweise Hydroxyethylmethacrylat.

Bevorzugte Comonomere sind Vinylacetat, Vinylpropionat und N-Vinylformamid, ganz besonders bevorzugt Vinylacetat.

Die Herstellung der Präpolymere erfolgt nach an sich bekannten Verfahren, z.B. durch Lösungs-, Fällungs-, oder durch umgekehrte Emulsions- Polymerisation in Anwesenheit von Verbindungen, die unter den Polymerisationsbedingungen Radikale bilden.

Die Polymerisationstemperaturen liegen üblicherweise in dem Bereich von 30 bis 200 °C, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Als Reaktionsmedium finden alle üblichen Lösungsmittel Verwendung, in denen die Monomere löslich sind. Vorzugsweise werden Wasser oder alkoholische Lösungsmittel wie z.B. Methanol, Ethanol, n-Propanol oder Isopropanol oder Gemische solcher Alkohole mit Wasser, eingesetzt.

Um zu gewährleisten, dass die Reaktion zu homogenen Produkten führen, ist es vorteilhaft, die Monomere und den Starter separat der Reaktionslösung zuzuführen. Dies kann beispielsweise in Form von getrennten Zuläufen für die einzelnen Reaktionspartner erfolgen.

Die Polymerisation kann auch in Gegenwart üblicher Regler durchgeführt werden, falls relativ niedrigere Molekulargewichte eingestellt werden sollen.

Ein für die Polymerisation verwendetes nicht-wässriges Lösungsmittel kann anschließend mittels Wasserdampfdestillation entfernt und gegen Wasser ausgetauscht werden.

Im Anschluss an die Copolymerisation erfolgt gegebenenfalls eine Verseifung unter Bildung der entprechenden Hydroxy- oder Amino-Funktionen. Die Verseifung kann in an sich bekannter Weise durch Umsetzung mit Basen wie beispielsweise alkoholische Lösungen von Hydroxiden wie Natrium- oder Kaliumhydroxid, oder alkoholische Lösungen von Alkoholaten wie beispielsweise methanolische Natrium- oder Kaliummethanolat-Lösung oder ethanolische Natrium- oder Kaliumethanolat-Lösung, durchgeführt werden.

Die so erhaltenen Präpolymere weisen üblicherweise Molekulargewichte Mw von 10.000 bis 150.000 g/mol auf.

Gemäß einer bevorzugten Ausführungsform der Erfindung werden die so erhaltenen Präpolymere mit Polyalkylenoxiden umgesetzt.

Als Polyalkylenoxide kommen vorzugsweise Polyalkylenglykole in Betracht. Die Polyalkylenglykole können Molekulargewichte von 500 bis 10.000 D [Dalton], vorzugsweise 1.000 bis 7.500 D, besonders bevorzugt 1.000 bis 5.000 D, aufweisen. Die Molekulargewichte werden ausgehend von der gemäß DIN 53240 gemessenen Hydroxyl-Zahl bestimmt.

Als besonders bevorzugte Polyalkylenglykole kommen Polyethylenglykole in Betracht. Weiterhin eignen sich auch Polypropylenglykole, Poly-Tetrahydrofurane oder Polybutylenglykole, die aus 2-Ethyloxiran oder 2,3-Dimethyloxiran erhalten werden..

Geeignete Polyether sind auch statistische oder blockartige Copolymere von aus Ethylenoxid, Propylenoxid und Butylenoxiden gewonnenen Polyalkylenglykolen wie beispielsweise Polyethylenglykol-polypropylenglykol-Blockcopolymere. Die Blockcopolymere können vom AB- oder vom ABA-Typ sein.

Zu den bevorzugten Polyalkylenglykolen gehören auch solche, die an einer der beiden OH-Endgruppen substituiert sind. Als Substituenten eignen sich Alkyl-, Aryl- oder Aralkyl-Reste mit 1 bis 30 C-Atomen. Geeignete Arylreste sind Phenyl-, Naphtyl-Reste geeignete Aralkylreste sind beispielsweise Benzylreste. Als Alkylreste kommen verzeigte oder unverzweigte, offenkettige oder cyclische C₁- bis C₂₂-Alkylreste in Betracht. Geeignete Cycloalkylreste sind beispielsweise Cyclopentyl-, Cyclohexyl-, Cycloheptyl- oder Cyclooctyl-Reste, diegegebenenfalls mit einem oder mehreren C1-C4-alkylresten substituiert sein können. Bevorzugt kommen C₁-C₁₈-Alkylreste, beispielsweise Methyl-, Ethyl-, n-Butyl-, Isobutyl-, Pentyl-, Hexyl-, Octyl-, Nonyl-, Decyl-, Dodecyl- Tridecyl- oder Octadecyl-Reste, in Betracht.

Solche Polyalkylenoxide sind entweder leicht herzustellen oder kommerziell erhältlich.

Die Polyalkylenoxide werden äquimolar, bezogen auf die Amino- oder Hydroxyl-Gruppen im Präpolymer, umgesetzt. Die Menge der vorhandenen OH- und Aminogruppen kann, soweit erforderlich, auf dem Fachmann an sich bekannte Weise erfolgen. Zur Ermittlung der OH-Zahl siehe beispielsweise Römpp Chemie Lexikon, 9. Auflage, 1990.

Gemäß einer Ausführungsform der Erfindung kann die Umsetzung der Vinyllactam-Copolymere mit Polyalkylenoxiden im Falle von hydroxylfunktionalisierten Vinyllactam-Copolymeren durch direkte Alkoxylierung der OH-Funktionen des Copolymeren erfolgen. Die direkte Alkoxilierung kann durch Umsetzung des Copolymeren mit dem entsprechenden monomeren Alkylenoxid in Gegenwart eines Katalysators erfolgen. Bevorzugt wird Kaliumhydroxid als Katalysator eingesetzt. Als Katalysatoren eignen sich weiterhin Alkalimetallhydroxide wie Natrium- oder Kaliumhydroxid, Montmorillonit, saure lonenaustauscher, Lewissäuren wie Bortrifluorid oder Doppelmetallcyanide wie Zink-Hexacyanoferrat Die Umsetzung kann in einem organischen Lösungmittel erfolgen, bevorzugt einem polaren aprotischen Lösungmittel, ganz besonders bevorzugt in Dimethylformamid. Die Umsetzung kann bei Temperaturen von 100 bis 180 °C erfolgen. Bevorzugt erfolgt die Umsetzung in einem Druckreaktor.

Eine weitere, bevorzugte, Ausführungsform der Erfindung betrifft die Kupplung von Vinyllactamcopolymeren und Polyalkylenoxiden durch Umsetzung mit Diisocyanaten. wobei es durch Reaktion mit den Hydroxyl- oder Amino-Gruppen des Vinyllactam-Copolymers zu einer Ankupplung des Polyalkylenoxids an das Vinyllactam-Copolymer über Urethan- oder Harnstoff-Gruppen kommt. Dabei kann entweder das Vinyllactam-Copolymer oder das Polyalkylenoxid zunächst mit dem Diisocyanat umgesetzt werden.

Gemäß einer bevorzugten Ausführungsform der Erfindung erfolgt die Ankupplung über mit Isocyanatgruppen als Endgruppen funktionalisierten Polyalkylenoxiden. Dazu wird zunächst das Polyalkylenoxid mit dem Diisocyanat umgesetzt und anschliessend das so funktionalisierte Polyalkylenoxid mit dem Copolymer umgesetzt.

Unabhängig davon, welche Ausführungsform der Erfindung gewählt wird, kann die Umsetzung wie folgt erfolgen:

Als Diisocyanate kommen Verbindungen der allgemeinen Formel OCN-R-NCO, wobei R aliphatische, alicyclische oder aromatische Reste, die auch mit Alkylresten substituiert sein können, bedeuten kann, in Betracht.

Als Diisocyanate eignen sich bevorzugt Verbindungen, deren Isocyanat-Gruppen aufgrund der Molekülstruktur eine unterschiedliche Reaktivität gegenüber Nucleophilen aufweisen, beispielsweise Isophorondiisocyanat oder Toluylendiisocyanat.

Grundsätzlich eignen sich auch symmetrische Diisocyanate wie beispielsweise Hexamethylendisocyanat oder 4,4'-Methylendi-(phenylisocyanat).

Bevorzugt wird Isophorondiisocyanat eingesetzt.

Die Umsetzung mit dem Diisocyanat erfolgt vorzugsweise in einem organischen Lösungsmittel wie Ketonen, beispielsweise Aceton, weiterhin Dimethylsulfoxid, Dimethylformamid, oder allgemein aprotisch-polaren organischen Lösungsmitteln oder Gemischen solcher Lösungsmittel. Die Umsetzung erfolgt üblicherweise bei erhöhten Temperaturen, wobei sich dieTemperatur auch nach der Siedetemperatur des gewählten Lösungsmittels richtet. Die Umsetzung des Diisocyanats mit der ersten Komponente kann bei 20 bis 50 °C, gewünschtenfalls aber auch bis 100 °C erfolgen. Die Umsetzung der zweiten Isocyanatgruppe kann bei Temperaturen von 50 bis 100 °C erfolgen.

Die Umsetzung erfolgt vorzugsweise äquimolar, was bedeutet, dass das Mengenverhältnis so gewählt wird, dass pro Mol umzusetzender Hydroxyl- oder Amino-Funktion vorzugsweise 1 Mol Diisocyanat eingesetzt werden.

Im Falle symmetrischer Diisocyanate kann es sich auch empfehlen, einen Überschuss an Diisocyanat einzusetzen und den Überschuss anschliessend destillativ zu entfernen.

Vorzugsweise wird die Umsetzung in Gegenwart eines Katalysators durchgeführt. Als Katalysatoren eignen sich beispielsweise metallorganische Verbindungen wie Organotitan-Verbindungen oder Zinkverbindungen wie Dibutylzinndilaurat oder Zinnoctoat, weiterhin Basen wie 1,4-Diaza(2,2,2)bicyclooctan oder Tetramethylbutandiamin.. Der Katalysator kann in Mengen von 0,05 bis 0,2 Mol, vorzugsweise 0,1 bis 0,1 Mol, pro Mol Diisocyanat eingesetzt werden.

Die Umsetzung wird üblicherweise bei erhöhten Temperaturen im Bereich von 50 bis 100 °C durchgeführt. Welche Temperatur im Einzelnen gewählt wird, hängt von der Art des verwendeten organischen Lösungsmittels ab. Das Lösungsmittel kann anschliessend durch Destillation entfernt werden.

Üblicherweise wird man die Umsetzung so durchführen, dass zunächst die Komponente, die isocyanatgruppenfunktionalisiert werden soll, mit dem Diisocyanat in Gegenwart des Katalysators und eines Lösungsmittels solange umgesetzt wird, bis der Isocyanat-Wert im Reaktionsgemisch auf die Hälfte gesunken ist. Dies lässt sich auf bekannte Weise beispielsweise titrimetrisch ermitteln. Danach erfolgt dann die Zugabe der anderen Komponente, wobei die Mengen an Isocyantgruppen und OH- oder Aminogruppen wiederum äquimolar gewählt werden. Die Reaktion wird fortgeführt, bis der Isocyanatwert auf Null gesunken ist.

Im Falle, dass nicht an alle Hydroxyl- oder Amino-Funktionen des Vinyllactam-Copolymers Polyalkylenoxide angekuppelt werden sollen, kann auch ein Unterschuss an isocyanatgruppenfunktionalisierten Polyalkylenoxiden eingesetzt werden.

Die so erhaltenen Copolymere auf Basis von Vinyllactam-Copolymeren und Polyalkylenoxiden sind wasserlöslich oder wasserdispergierbar. Bevorzugt sind wasserlösliche Copolymere.

Die Molekulargewichte M_{w} können 20.000 bis 500.000 g/mol betragen; vorzugsweise 20.000 bis 250.000, besonders bevorzugt 80.000 bis 250.000 g/mol. Die Molekulargewichte können mit Hilfe der Gelchromatographie bestimmt werden.

### Anwendungen:

Die erfindungsgemäß zu verwendenden Copolymere lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen. Die Copolymere finden demgemäß Verwendung als Solubilisatoren von in Wasser schwerlöslichen Substanzen, insbesondere biologisch aktiven Substanzen.

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Bei praktisch unlöslichen Substanzen werden mindestens 10.000 g Wasser pro g Substanz benötigt.

Im Sinne der vorliegenden Erfindung sind unter in Wasser schwerlösliche biologisch aktive Substanzen pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

Durch die vorliegende Erfindung werden insbesondere amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe zu solubilisieren.

### Solubilisatoren für Kosmetik:

Erfindungsgemäßen können die Copolymere als Solubilisatoren in kosmetischen Formulierungen eingesetzt werden. Beispielsweise eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkiefernöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Weiterhin können die erfindungsgemäßen Polymere als Solubilisatoren für in Wasser schwerlösliche oder unlösliche UV-Absorber wie beispielsweise 2-Hydroxy-4-methoxybenzophenon (Uvinul® M 40, Fa. BASF), 2,2',4,4'-Tetrahydroxybenzophenon (Uvinul® D 50), 2,2'-Dihydroxy-4,4'-dimethoxybenzophenon (Uvinul®D49), 2,4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul^{®} T 150), 3-(4-Methoxybenzyliden)campher (Eusolex^{®} 6300, Fa. Merck), N,N-Dimethyl-4-aminobenzoesäure-2-ethylhexylester (Eusolex^{®} 6007), Salicylsäure-3,3,5-trimethylcyclohexylester, 4-lsopropyl-dibenzoylmethan (Eusolex® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens einen der erfindungsgemäßen Copolymere der eingangs genannten Zusammensetzung als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol Basis. Die erfindungsgemäßen Solubilisatoren werden im Verhältnis von 0,2:1 bis 20:1, bevorzugt 1:1 bis 15:1, besonders bevorzugt 2:1 bis 12:1 zum schwerlöslichen kosmetischen Wirkstoff eingesetzt.

Der Gehalt an erfindungsgemäßem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholethersulfate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Haarwässern, Eau de Cologne, Eau de Toilette sowie in Sonnenschutzmitteln Verwendung. Ein weiteres Einsatzgebiet für Polymere dieser Art ist der Bereich Oral Care, beispielsweise in Mundwässern, Zahnpasten, Haftcremes für Zahnprothesen und dergleichen.

Weiterhin eignen sich die Copolymerisate auch für technische Anwendungen, beispielsweise für Zubereitungen von schwerlöslichen Farbmitteln, in Tonern, Zubereitungen von Magnetpigmenten und dergleichen.

### Beschreibung der Solubilisierungsmethode:

Bei der Herstellung der Solubilisate für kosmetische Formulierugen können die erfindungsgemäßen Copolymere als 100%ige Substanz oder bevorzugt als wäßrige Lösung eingesetzt werden.

Üblicherweise wird der Solubilisator in Wasser gelöst und mit dem jeweils zu verwendenden schwerlöslichen kosmetischen Wirkstoff intensiv vermischt.

Es kann aber auch der Solubilisator mit dem jeweils zu verwendenden schwerlöslichen kosmetischen Wirkstoff intensiv vermischt werden und anschließend unter ständigem Rühren mit demineralisiertem Wasser versetzt werden.

### Solubilisatoren für pharmazeutische Anwendungen:

Die beanspruchten Copolymerisate eignen sich ebenso für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe sowie Vitamine und/oder Carotinoide enthalten können. Insbesondere handelt es sich dabei um wäßrige Lösungen bzw. Solubilisate zur oralen Applikation. So eignen sich die beanspruchten Copolymere zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die beanspruchten Copolymere um einen schwerlöslichen Arzneistoff zu verarbeiten.

Pharmazeutische Formulierungen der oben genannten Art können durch Verarbeiten der beanspruchten Copolymere mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolvenzien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, Antibiotika, Antimykotika, antivirale Mittel, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Eine mögliche Herstellvariante ist das Auflösen des Solubilisators in der wässrigen Phase, gegebenenfalls unter leichtem Erwärmen und das anschließende Lösen des Wirkstoffs in der wässrigen Solubilisatorlösung. Das gleichzeitige Auflösen von Solubilisator und Wirkstoff in der wäßrigen Phase ist ebenfalls möglich.

Die Verwendung der erfindungsgemäßen Copolymere als Solubilisator kann beispielsweise auch in der Weise erfolgen, daß der Wirkstoff in dem Solubilisator, gegebenenfalls unter Erwärmen, dispergiert wird und unter Rühren mit Wasser vermischt wird.

Weiterhin können die Solubilisatoren auch in der Schmelze mit den Wirkstoffen verarbeitet werden. Insbesondere können auf diese Weise feste Lösungen erhalten werden. Hierfür eignet sich unter anderem auch das Verfahren der Schmelzextrusion. Eine weitere Möglichkeit zur Herstellung von festen Lösungen ist auch, Lösungen von Solubilisator und Wirkstoff in geeigneten organischen Lösungsmitteln herzustellen und das Lösungsmittel anschliessend durch übliche Verfahren zu entfernen.

Gegenstand der Erfindung sind daher auch allgemein pharmazeutische Zubereitungen, die mindestens einen der erfindungsgemäßen Copolymere als Solubilisator enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um oral applizierbare Formulierungen handelt.

Der Gehalt an erfindungsgemäßem Solubilisator in der pharmazeutischen Zubereitung liegt, abhängig vom Wirkstoff, im Bereich von 1 bis 75 Gew.-%, bevorzugt 5 bis 60 Gew.-%, besonders bevorzugt 5 bis 50 Gew.-%.

Im Falle von festen Lösungen kann das Gewichtsverhältnis von Solubilisator zu Wirkstoff von 1:1 bis 4:1 betragen.

### Solubilisatoren für Lebensmittelzubereitungen:

Neben der Anwendung in der Kosmetik und Pharmazie eignen sich die erfindungsgemäßen Copolymeren auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiele seien klare, mit Carotinoiden gefärbte Getränke genannt.

### Solubilisatoren für Pflanzenschutzzubereitungen:

Die Anwendung der erfindungsgemäßen Copolymere als Solubilisatoren in der Agrochemie kann u.a. Formulierungen umfassen, die Pestizide, Herbizide, Fungizide oder Insectizide enthalten, vor allem auch solche Zubereitungen von Pflanzenschutzmitteln, die als Spritz- oder Gießbrühen zum Einsatz kommen.

Die erfindungsgemäßen Copolymere zeichnen sich durch eine besonders gute solubilisierende Wirkung aus. Vorteilhaft ist auch, dass es sich um Feststoffe handelt, die verarbeitungstechnisch günstigere Eigenschaften aufweisen.

In den folgenden Beispielen wird die Herstellung und Verwendung der erfindungsgemäßen Copolymere näher erläutert.

### Herstellung der Vinyllactam-Präpolymere

### VP: N-Vinylpyrrolidon; VAc: Vinylacetat

### Allgemeine Vorschrift

Vorlage: 10 g von Zulauf 1, 200 g Methanol
Zulauf 1: VP, VAc
Zulauf 2: tert-Butylperpivalat (75 gew.%-ig in Aliphatengemisch),67 g Methanol
Zulauf 3: tert-Butylperpivalat (75 gew.%-ig in Aliphatengemisch), 200 g Methanol
Zulauf 4: Natriummethylat, 5 g Methanol
Genaue Mengenangaben siehe nachstehende Tabelle

Die Herstellung erfolgte in einer Rührapparatur unter einer Stickstoffatmosphäre. Die Vorlage wurde auf 65 °C erwärmt, mit 3,4 g von Zulauf 2 versetzt und 15 min polymerisiert. Dann wurden Zulauf 1 und die Restmenge von Zulauf 2 gestartet und über einen Zeitraum von 4 stunden zugegeben. Anschliessend wurde Zulauf 3 als Batch zugegeben und für zwei Stunden nachpolymerisiert. Danach wurde Zulauf 4 bei 58 °C zugegeben und das Reaktionsgemisch eine Stunde bei dieser Temperatur gehalten. Methanol und entstandenes Methylacetat wurden abdestilliert und das Produkt einer Wasserdampfdestillation unterworfen. Nach der Wasserdampfdestillation wurde eine klare gelbliche Lösung erhalten, aus der das Copolymer durch Gefriertrocknung erhalten wurde.

| Präpolymer | | Mengen Einsatzstoffe [g] | | | | | K-Wert 1 Gew.-%ig in Wasser |
|---|---|---|---|---|---|---|---|
| Bsp.nr | VP:VAc [mol-%] | VP | VAc | NaOMe | Initiator | | |
| | | | | | Zul.2 | Zul.3 | |
| A | 98:2 | 196 | 4 | 0,14 | 1,33 | 1,33 | 49,1 |
| B | 96:4 | 192 | 8 | 0,27 | 1,33 | 1,33 | 52 |
| C | 98:2 | 196 | 4 | 0,14 | 2,66 | 1,33 | 43,2 |
| D | 96:4 | 192 | 8 | 0,27 | 2,66 | 1,33 | 41,8 |

Die Molekulargewichtsbestimmung erfolgte über Größenausschlusschromatographie mit Dimethylacetamid + 0,5 Gew% LiBr als Lösungsmittel (Temperatur: 80°C, Flussrate: 1 ml/min, Konzentration der Lösung: 5 g/l). Die GPC-Säule wurde mittels PMMA-Standard (M=800 - 1.820.000 g/mol) kalibriert.

| | M_{w} [g/mol] | Mₙ [g/mol] | M_{w}/Mₙ |
|---|---|---|---|
| Präpolymer A | 145.000 | 21.000 | 6.9 |
| Präpolymer B | 170.000 | 25.000 | 6.8 |
| Präpolymer C | 87.000 | 14.000 | 6.2 |
| Präpolymer D | 79.000 | 15.000 | 5.3 |

### Herstellung der Copolymere: Beispiele 1 bis 16

### Allgemeine Vorschrift

25 mmol Isophorondiisocyanat, 200 g Lösungmittel und 5 mmol Dibutylzinndilaurat wurden vorgelegt und auf die gewünschte Temperatur gebracht. Anschliessend wurden 25 mmol, bezogen auf OH-Gruppen, des Polyalkylenoxids zugegeben und das Gemisch solange bei der eingestellten Temperatur gehalten, bis der Isocyanatwert auf 50 % gesunken ist. Danach wurden 25 mmol, bezogen auf OH-Gruppen, des Vinyllactam-Präpolymers zugegeben und die Temperatur auf die gewählte Reaktionstemperatur erhöht. ,

Die Bestimmung der Hyrdoxyl-Zahlen der Polyalkylenoxide und Vinyllactampräpolymere erfolgt durch Acetylierung der Hydroxyl-Gruppen mit Essigsäureanhydrid und anschliessender Titration der entstandenen Essigsäure mit Base (DIN 53240 und DIN 16945, siehe Römpp, 9.Auflage).

Die Bestimmung des Isocyanatwerts erfolgte titrimetrisch: 1 g des Produktes wurden in 20 ml einer 0,1 molaren Lösung von Dibutylamin in Toluol gelöst und unter Verwendung von Bromphenolblau als Indikator mit 0,1 molarer Salzsäure zurücktitriert.

| Bsp.nr. | Eingesetztes Polyalkylenoxid | Lösungsmittel | Temp. IPDI-Ums. [°C] | Vinyllactam-Präpolymer |
|---|---|---|---|---|
| 1 | Kerocom 3271 | Aceton/DMSO | 57 | C |
| 2 | Pluronic PE 3100 | Aceton/DMSO | 57 | C |
| 3 | Pluriol A 2000 E | DMSO | 100 | C |
| 4 | Kerocom 3271 | DMSO | 100 | D |
| 5 | Pluronic PE 3100 | DMSO | 100 | D |
| 6 | Pluriol A 1350 P | DMSO | 20 | C |
| 7 | Pluriol P 2000 | DMSO | 100 | D |
| 8 | Pluriol A 1350 P | DMSO | 20 | D |
| 9 | Pluriol P 2000 | DMF | 100 | C |
| 10 | Pluriol P 2000 | DMF | 100 | D |
| 11 | Pluronic PE 6100 | Aceton | 57 | C |
| 12 | Pluronic PE 6100 | Aceton | 57 | D |
| 13 | Pluronic PE 6200 | Aceton | 57 | C |
| 14 | Pluronic PE 6200 | Aceton | 57 | D |
| 15 | Pluronic PE 10100 | Aceton | 57 | C |
| 16 | Pluronic PE 10100 | Aceton | 57 | D |

### Verwendete Polyalkylenoxide (AlkO)

| | | |
|---|---|---|
| Pluriol | A 1350 P | 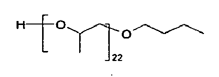 |
| | A 2000 E | 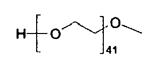 |
| | P 2000 | 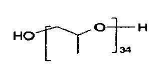 |
| Pluronic | PE 3100 | 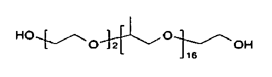 |
| | PE 6100 | 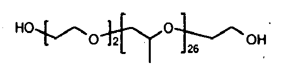 |
| | PE 6200 | 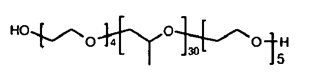 |
| | PE 10100 | 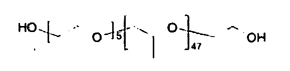 |
| Kerocom | 3271 | 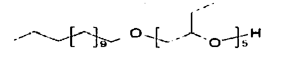 |

| Beispiel Nr. | Bruttozusammensetzung in mol-%, bez. auf Einsatzmengen | | | | Mol.w. bestimmt durch GPC |
|---|---|---|---|---|---|
| | VP | VAc | IPDI | AlkO | |
| 1 | 83,1 | 1,69 | 1,69 | 13,52 | 103.000 |
| 2 | 81,03 | 1,65 | 1,65 | 15,67 | 200.000 |
| 3 | 72,11 | 1,47 | 1,47 | 24,95 | 215.000 |
| 4 | 70,66 | 2,93 | 2,93 | 23,48 | 91.000 |
| 5 | 67,68 | 2,81 | 2,81 | 26,70 | 174.000 |
| 6 | 66,27 | 1,35 | 1,35 | 31,03 | 113.000 |
| 7 | 55,90 | 2,32 | 2,32 | 39,46 | 197.000 |
| 8 | 49,06 | 2,04 | 2,04 | 46,86 | 107.000 |
| 9 | 72,12 | 1,47 | 1,47 | 24,94 | 175.000 |
| 10 | 55,88 | 2,32 | 2,32 | 39,48 | 228.000 |
| 11 | 74,87 | 1,52 | 1,52 | 23,09 | 171.000 |
| 12 | 59,33 | 2,46 | 2,46 | 35,75 | 219.000 |
| 13 | 69,57 | 1,42 | 1,42 | 27,59 | 177.000 |
| 14 | 52,82 | 2,19 | 2,19 | 42,80 | 233.000 |
| 15 | 63,3 | 1,29 | 1,29 | 34.02 | 188.000 |
| 16 | 45,79 | 1,9 | 1,9 | 50,41 | 258.000 |

### Herstellung von Solubilisaten

In ein Becherglas wurden 2g des Copolymers eingewogen. Anschließend wurde dem Ansatz jeweils ein Arzneistoff wie folgt zugewogen, um eine übersättigte Lösung zu erhalten. (Falls sich die eingewogene Masse im Medium auflöste, wurde die Einwaage bis zur Ausbildung eines Bodensatzes erhöht).
Zugewogene Menge an Wirkstoff: 17-β-Estradiol 0,2 g; Piroxicam 0,2 ; Clotrimazol 0,2 g; Carbamazepin 0,3 g; Ketoconazol 0,25 g; Griseofulvin 0,25 g; Cinnarizin 0,25 g.

Anschließend wurde Phosphatpuffer pH 7,0 hinzugegeben, bis Solubilisator und Phosphatpuffer im Gewichtsverhältnis von 1:10 vorlagen. Mit Hilfe eines Magnetrührers wurde dieser Ansatz bei 20°C 72 Stunden gerührt. Danach erfolgte mindestens eine 1 stündige Ruhezeit. Nach der Filtration des Ansatzes wurde dieser photometrisch vermessen und der Gehalt an Wirkstoff bestimmt.

| | | | | | | | | |
|---|---|---|---|---|---|---|---|---|
| Carbamazepin | Copolymer gemäß Bsp.nr. / Solubilisierung bei 20 °C in [g/100 ml] | | | | | | | |
| | 1/ 0.08 | 2/ 0.07 | 3/ 0.07 | 4/ 0.08 | 5/ 0.08 | 6/ 0.09 | 7/ 0.11 | 8/ 0.20 |

| | | | | |
|---|---|---|---|---|
| Estradiol | Copolymer gemäß Bsp.nr. / Solubilisierung bei 20 °C in [g/100 ml] | | | |
| | 1/ 0.01 | 3/0.05 | 5/0.02 | 6/0.16 |

| | | | | | | | | |
|---|---|---|---|---|---|---|---|---|
| Piroxicam | Copolymer gemäß Bsp.nr. / Solubilisierung bei 20 °C in [g/100 ml] | | | | | | | |
| | 1/ 0.29 | 2/ 0.48 | 3/ 0.47 | 4/ 0.32 | 5/ 0.29 | 6/ 0.33 | 7/ 0.39 | 8/ 0.36 |

## Patentansprüche

1. Mit Polyalkylenoxid-Seitenketten modifizierte Vinyllactam-Copolymere, wobei die Herstellung der Copolymere durch radikalisch initiierte Copolymerisation von N-Vinyllactamen mit monoolefinisch ungesättigten Comonomeren, die Hydroxyl-oder Aminogruppen tragen oder eine durch Hydrolyse in Hydroxyl-oder Aminogruppen überführbare Gruppe enthalten, und anschliessender Ankupplung der Polyalkylenoxid-Seitenketten an die Amino- oder HydroxyGruppen der Vinyllactam-Copolymere erfolgt.

2. Copolymere nach Anspruch 1, wobei als monoolefinisch ungesättigte Comonomere Monomere ausgewählt aus der Gruppe bestehend aus Vinylestern von C₂-C₈-Carbonsäuren, Monovinylethern von C₂-C₈-Diolen, Vinylamiden, Hydroxyalkyl- und Aminoalkylestern der Acrylsäure und der Methacrylsäure.

3. Copolymere nach einem der Ansprüche 1 oder 2, wobei die Vinyllactam-Copolymere durch radikalisch initiierte Copolymerisation von N-Vinyllactamen und Vinylacetat mit anschliessender Verseifung der Estergruppen erhalten werden.

4. Copolymere nach einem der Ansprüche 1 bis 3, wobei als N-Vinyllactam N-Vinylpyrrolidon eingesetzt wird.

5. Copolymere nach einem der Ansprüche 1 bis 4, wobei das Vinyllactam-Copolymer durch Umsetzung von 50 bis 99,9 mol-% N-Vinyllactamen und 0,1 bis 50 mol-% Comonomeren erhalten wird.

6. Copolymere nach einem der Ansprüche 1 bis 5, wobei das Vinyllactam-Copolymer durch Umsetzung von 80 bis 99 mol-% N-Vinyllactamen und 1 bis 20 mol-% Comonomeren erhalten wird.

7. Copolymere nach einem der Ansprüche 1 bis 6, wobei die Polyalkylenoxide Molekulargewichte von 500 bis 20.000 Dalton aufweisen.

8. Copolymere nach einem der Ansprüche 1 bis 7, wobei die Polyalkylenoxide ausgewählt sind aus der Gruppe bestehend aus Polyethylenglykolen, Polypropylenglykolen, Polybutylenglykolen und deren monoalkoxilierten Derivaten.

9. Copolymere nach einem der Ansprüche 1 bis 7, wobei als Polyalkylenoxide Polyoxyethylen-polyoxypropylen-Blockcopolymere eingesetzt werden

10. Copolymere nach einem der Ansprüche 1 bis 9, wobei die Ankupplung der Polyalkylenoxid-Seitenketten über Diisocyanate erfolgt.

11. Copolymere nach einem der Ansprüche 1 bis 10, wobei die Polyalkylenoxide vor der Umsetzung mit dem Vinyllactam-Copolymer durch Umsetzung mit einem Diisocyanat funktionalisiert werden.

12. Copolymere nach einem der Ansprüche 1 bis 11, wobei die Polyalkylenoxide in äquimolaren Mengen, bezogen auf die Amino- oder Hydroxyl-Gruppen des Vinyllactam-Copolymeren, eingesetzt werden.

13. Copolymere nach einem der Ansprüche 1 bis 12 , wobei Diisocyanat Isophorondiisocyanat eingesetzt wird.

14. Copolymere nach einem der Ansprüche 1 bis 6, wobei als die Ankupplung der Polyalkylenoxid-seitenketten durch direkte Alkoxilierung mit Alkylenoxiden erfolgt.

15. Copolymere einem der Ansprüche 1 bis 14, wobei die Copolymeren Molekulargewichte M_{w} von 20.000 bis 250.000 g/mol aufweisen.

16. Verwendung von Copolymeren nach einem der Ansprüche 1 bis 14 als Solubilisatoren für in Wasser schwerlösliche Substanzen.

17. Verwendung nach Anspruch 16, wobei es sich bei den in Wasser schwerlöslichen Substanzen um biologisch aktive Substanzen handelt.

18. Verwendung nach Anspruch 16 oder 17, zur Herstellung von pharmazeutischen Zubereitungen für die Behandlung von Krankheiten

19. Verwendung nach Anspruch 16 oder 17 für kosmetische Zubereitungen.

20. Verwendung nach Anspruch 16 oder 17 für agrochemische Zubereitungen.

21. Verwendung nach Anspruch 16 oder 17 für Nahrungsergänzungsmittel oder dietätische Mittel.

22. Verwendung nach Anspruch 16 oder 17 für Lebensmittel.

23. Verwendung nach Anspruch 16 für Zubereitungen von Farbstoffen.

24. Zubereitungen von in Wasser schwerlöslichen Substanzen, enthaltend als Solubilisatoren Copolymere nach einem der Ansprüche 1 bis 15.

25. Zubereitungen nach Anspruch 24, enthaltend als in Wasser schwerlösliche Substanz eine biologisch aktive Substanz.

26. Zubereitungen nach Anspruch 24, enthaltend als in Wasser schwerlösliche biologisch aktive substanz einen pharmazeutischen Wirkstoff.

27. Zubereitungen nach Anspruch 26 in Form oral applizierbarer Darreichungsformen.

28. Zubereitungen nach Anspruch 24 oder 25 enthaltend als in Wasser schwerlösliche biologisch aktive Substanz einen kosmetischen Wirkstoff.

29. Zubereitungen nach Anspruch 24 oder 25 enthaltend als in Wasser schwerlösliche biologisch aktive Substanz einen agrochemischen Wirkstoff.

30. Zubereitungen nach Anspruch 24 oder 25, enthaltend als in Wasser schwerlösliche biologisch aktive Substanz ein Nahrungsergänzungsmittel oder einen dietätischen Wirkstoff.

31. Zubereitungen nach Anspruch 24, enthaltend als in Wasser schwerlösliche Substanz einen Farbstoff.

## Claims

1. A vinyllactam copolymer modified with polyalkylene oxide side-chains, where the copolymer is produced by free-radically initiated copolymerization of N-vinyllactams with monoolefinically unsaturated comonomers which carry hydroxyl or amino groups or comprise a group which can be converted into hydroxyl or amino groups by hydrolysis, and subsequent coupling of the polyalkylene oxide side-chains onto the amino or hydroxy groups of the vinyllactam copolymer.

2. The copolymer according to claim 1, where the monoolefinically unsaturated comonomers monomers chosen from the group consisting of vinyl esters of C₂-C₈-carboxylic acids, monovinyl ethers of C₂-C₈-diols, vinylamides, hydroxyalkyl and aminoalkyl esters of acrylic acid and of methacrylic acid.

3. The copolymer according to either of claims 1 and 2, where the vinyllactam copolymers are obtained by free-radically initiated copolymerization of N-vinyllactams and vinyl acetate with subsequent saponification of the ester groups.

4. The copolymer according to one of claims 1 to 3, where the N-vinyllactam used is N-vinylpyrrolidone.

5. The copolymer according to one of claims 1 to 4, where the vinyllactam copolymer is obtained by reacting 50 to 99.9 mol% of N-vinyllactams and 0.1 to 50 mol% of comonomers.

6. The copolymer according to one of claims 1 to 5, where the vinyllactam copolymer is obtained by reacting 80 to 99 mol% of N-vinyllactams and 1 to 20 mol% of comonomers.

7. The copolymer according to one of claims 1 to 6, where the polyalkylene oxides have molecular weights of from 500 to 20,000 daltons.

8. The copolymer according to one of claims 1 to 7, where the polyalkylene oxides are chosen from the group consisting of polyethylene glycols, polypropylene glycols, polybutylene glycols and monoalkoxylated derivatives thereof.

9. The copolymer according to one of claims 1 to 7, where the polyalkylene oxides used are polyoxyethylene-polyoxypropylene block copolymers.

10. The copolymer according to one of claims 1 to 9, where the coupling of the polyalkylene oxide side-chains takes place via diisocyanates.

11. The copolymer according to one of claims 1 to 10, where the polyalkylene oxides are functionalized by reaction with a diisocyanate before reaction with the vinyllactam copolymer.

12. The copolymer according to one of claims 1 to 11, where the polyalkylene oxides are used in equimolar amounts, based on the amino or hydroxyl groups of the vinyllactam copolymer.

13. The copolymer according to one of claims 1 to 12, where the diisocyanate used is isophorone diisocyanate.

14. The copolymer according to one of claims 1 to 6, where as the coupling of the polyalkylene oxide side-chains takes place by direct alkoxylation with alkylene oxides.

15. The copolymer one of claims 1 to 14, where the copolymers have molecular weights M_{w} of from 20,000 to 250,000 g/mol.

16. The use of copolymers according to one of claims 1 to 14 as solubilizers for substances which are sparingly soluble in water.

17. The use according to claim 16, where the substances which are sparingly soluble in water are biologically active substances.

18. The use according to claim 16 or 17, for producing pharmaceutical preparations for the treatment of illnesses.

19. The use according to claim 16 or 17 for cosmetic preparations.

20. The use according to claim 16 or 17 for agrochemical preparations.

21. The use according to claim 16 or 17 for food supplements or dietetic agents.

22. The use according to claim 16 or 17 for foods.

23. The use according to claim 16 for preparations of dyes.

24. A preparation of substances which are sparingly soluble in water, comprising, as solubilizers, copolymers according to one of claims 1 to 15.

25. The preparation according to claim 24, comprising a biologically active substance as substance which is sparingly soluble in water.

26. The preparation according to claim 24, comprising a pharmaceutical active ingredient as biologically active substance which is sparingly soluble in water.

27. The preparation according to claim 26 in the form of orally applicable administration forms.

28. The preparation according to claim 24 or 25, comprising a cosmetic active ingredient as biologically active substance which is sparingly soluble in water.

29. The preparation according to claim 24 or 25, comprising an agrochemical active ingredient as biologically active substance which is sparingly soluble in water.

30. The preparation according to claim 24 or 25, comprising a food supplement or a dietetic active ingredient as biologically active substance which is sparingly soluble in water.

31. The preparation according to claim 24, comprising a dye as substance which is sparingly soluble in water.

## Revendications

1. Copolymères de vinyllactame modifiés avec des chaînes latérales polyoxyalkylène, la préparation des copolymères s'effectuant par copolymérisation à amorçage radicalaire de N-vinyllactames avec des comonomères à insaturation mono-oléfinique, qui portent des groupes hydroxy ou amino ou contiennent un groupe pouvant être converti par hydrolyse en groupes hydroxy ou amino, et couplage subséquent des chaînes latérales polyoxyalkylène aux groupes amino ou hydroxy des copolymères de vinyllactame.

2. Copolymères selon la revendication 1, dans lesquels en tant que comonomères à insaturation mono-oléfinique on utilise des monomères choisis dans le groupe constitué par des esters vinyliques d'acides carboxyliques en C₂-C₈, des éthers monovinyliques de diols en C₂-C₈, des vinylamides, des esters hydroxyalkyliques et aminoalkyliques de l'acide acrylique et de l'acide méthacrylique.

3. Copolymères selon la revendication 1 ou 2, dans lesquels les copolymères de vinyllactame sont obtenus par copolymérisation à amorçage radicalaire de N-vinyllactame et acétate de vinyle avec saponification subséquente des groupes ester.

4. Copolymères selon l'une quelconque des revendications 1 à 3, dans lesquels on utilise comme N-vinyllactame la N-vinylpyrrolidone.

5. Copolymères selon l'une quelconque des revendications 1 à 4, dans lesquels le copolymère de vinyllactame est obtenu par réaction de 50 à 99,9 % en moles de N-vinyllactame et 0,1 à 50 % en moles de comonomères.

6. Copolymères selon l'une quelconque des revendications 1 à 5, dans lesquels le copolymère de vinyllactame est obtenu par réaction de 80 à 99 % en moles de N-vinyllactame et 1 à 20 % en moles de comonomères.

7. Copolymères selon l'une quelconque des revendications 1 à 6, dans lesquels les polyoxyalkylènes présentent des masses moléculaires de 500 à 20 000 daltons.

8. Copolymères selon l'une quelconque des revendications 1 à 7, dans lesquels les polyoxyalkylènes sont choisis dans le groupe constitué par les polyéthylèneglycols, les polypropylèneglycols, les polybutylèneglycols et leurs dérivés monoalcoxylés.

9. Copolymères selon l'une quelconque des revendications 1 à 7, dans lesquels on utilise comme polyalkylèneglycols des copolymères séquences polyoxyéthylène-polyoxypropylène.

10. Copolymères selon l'une quelconque des revendications 1 à 9, dans lesquels le couplage des chaînes latérales polyoxyalkylène s'effectue par l'intermédiaire de diisocyanates.

11. Copolymères selon l'une quelconque des revendications 1 à 10, dans lesquels les polyoxyalkylènes sont fonctionnalisés, avant la réaction avec le copolymère de vinyllactame, par réaction avec un diisocyanate.

12. Copolymères selon l'une quelconque des revendications 1 à 11, dans lesquels les polyoxyalkylènes sont utilisés en quantités équimolaires, par rapport aux groupes amino ou hydroxy du copolymère de vinyllactame.

13. Copolymères selon l'une quelconque des revendications 1 à 12, dans lesquels on utilise le diisocyanate isophorone-diisocyanate.

14. Copolymères selon l'une quelconque des revendications 1 à 6, dans lesquels le couplage des chaînes latérales polyoxyalkylène s'effectue par alcoxylation directe avec des oxydes d'alkylène.

15. Copolymères selon l'une quelconque des revendications 1 à 14, dans lesquels les copolymères présentent des masses moléculaires M_{w} de 20 000 à 250 000 g/mole.

16. Utilisation des copolymères selon l'une quelconque des revendications 1 à 14, en tant qu'agent solubilisant pour des substances difficilement solubles dans l'eau.

17. Utilisation selon la revendication 16, dans laquelle les substances difficilement solubles dans l'eau consistent en des substances biologiquement actives.

18. Utilisation selon la revendication 16 ou 17, pour la fabrication de préparations pharmaceutiques pour le traitement de maladies.

19. Utilisation selon la revendication 16 ou 17, pour des préparations cosmétiques.

20. Utilisation selon la revendication 16 ou 17, pour des préparations agrochimiques.

21. Utilisation selon la revendication 16 ou 17, pour des compléments nutritionnels ou des produits diététiques.

22. Utilisation selon la revendication 16 ou 17, pour des produits alimentaires.

23. Utilisation selon la revendication 16, pour des compositions de colorants.

24. Préparations de substances difficilement solubles dans l'eau, contenant en tant qu'agents solubilisants des copolymères selon l'une quelconque des revendications 1 à 15.

25. Préparations selon la revendication 24, contenant en tant que substance difficilement soluble dans l'eau une substance biologiquement active.

26. Préparations selon la revendication 24, contenant en tant que substance difficilement soluble dans l'eau une substance active pharmaceutique.

27. Préparations selon la revendication 26, sous forme de formes galéniques pouvant être administrées par voie orale.

28. Préparations selon la revendication 24 ou 25, contenant en tant que substance biologiquement active difficilement soluble dans l'eau une substance active cosmétique.

29. Préparations selon la revendication 24 ou 25, contenant en tant que substance biologiquement active difficilement soluble dans l'eau une substance active agrochimique.

30. Préparations selon la revendication 24 ou 25, contenant en tant que substance biologiquement active difficilement soluble dans l'eau un complément nutritionnel ou une substance active diététique.

31. Préparations selon la revendication 24, contenant en tant que substance difficilement soluble dans l'eau un colorant.
